# EUROPÄISCHE PATENTSCHRIFT

(11) **EP 3 697 348 B1**
(45) Veröffentlichungstag und Bekanntmachung des Hinweises auf die Patenterteilung: **29.12.2021**
(21) Anmeldenummer: 18783467.6
(22) Anmeldetag: 08.10.2018
(51) Int. Cl.: A61F 2/64, A61F 2/68, A61F 2/50, A61F 2/74

(54) **ORTHOPÄDIETECHNISCHES GELENK**
ORTHOPEDIC JOINT
ARTICULATION ORTHOPÉDIQUE

(30) Priorität: 18.10.2017 DE 102017124337
(43) Veröffentlichungstag der Anmeldung: 26.08.2020
(73) Patentinhaber: Ottobock SE & Co. KGaA, 37115 Duderstadt (DE)
(72) Erfinder: BOITEN, Herman, 6715 LE Ede (NL)
(74) Vertreter: Gramm, Lins & Partner Patent- und Rechtsanwälte PartGmbB
(86) Internationale Anmeldenummer: PCT/EP2018/077317
(87) Internationale Veröffentlichungsnummer: WO 2019/076664

(56) Entgegenhaltungen:
- WO-A1-99/00075
- DE-A1-102005 029 160
- US-A- 5 704 945
- US-B1- 9 913 738

## Beschreibung

Die Erfindung betrifft ein orthopädietechnisches Gelenk mit einem Oberteil, einem daran um eine Schwenkachse schwenkbar gelagerten Unterteil und eine Rotationshydraulik, die ein Schwenkgehäuse mit einer Kammer und einem darin schwenkbar gelagerten Schwenkkolben aufweist, der die Kammer in eine Flexionskammer und eine Extensionskammer unterteilt, die über zumindest einen Kanal hydraulisch miteinander verbunden sind, und mit einer Vorspanneinrichtung, die eine Verschwenkbewegung des Oberteils relativ zu dem Unterteil unterstützt, wie in den Ansprüchen definiert.

Orthopädietechnische Gelenke für orthopädietechnische Einrichtungen wie Orthesen oder Exoprothesen dienen dazu, ein Oberteil gelenkig mit einem Unterteil zu verbinden. Zwischen dem Oberteil und dem Unterteil wird eine Schwenkachse gebildet, die bei einem monozentrischen Gelenk eine feste Zuordnung zu dem Oberteil und dem Unterteil hat, bei einem polyzentrischen Gelenk kann die Schwenkachse sich relativ zu dem Oberteil oder dem Unterteil über den Schwenkwinkel verändern.

Aus der DE 297 23 632 U1 ist eine computergesteuerte hydraulische Widerstandseinrichtung für eine Prothese mit einem Prothesenschaft für einen Oberschenkelstumpf, einer Kniesteuerungsanordnung mit einer Widerstandseinrichtung, einem Unterschenkelrohr und einem Prothesenfuß bekannt. Ein Oberteil in Gestalt einer Knieklammer weist eine Rotorwelle auf, an der ein Flügel angeordnet ist. Über die Rotorwelle lässt sich die Knieklammer zusammen mit dem Flügel relativ zu einem Rahmen verlagern, der das Unterteil ausbildet und eine Aufnahme für ein Unterschenkelrohr aufweist. Innerhalb des Rahmens ist ein Gehäuse ausgebildet, in dem der Flügel verschwenkt wird, sodass sich eine Rotationshydraulik ausbildet. Über ein Verbindungskanal, in dem Drosseln und Ventile angeordnet sind, wird während der Rotation das Hydraulikfluid von einer Extensionskammer in eine Flexionskammer geleitet und umgekehrt. In dem Fluidstrom ist eine Kammer angeordnet, in der ein Kolben und eine Feder angeordnet sind, über eine vollständige Extension des Prothesenkniegelenkes sichergestellt wird.

Die EP 1 736 121 B1 betrifft eine hydraulische Kniegelenksprothese mit einer Gelenkeinheit, einer Fußanschlusseinheit und einem die beiden Komponenten verbindenden Rohr, wobei die Gelenkeinheit eine Drehachse mit einer Dämpferkammer und einem darin bewegbaren Dämpferflügel beinhaltet, die von einer Hydrauliksteuerung angesteuert ist. Die Fußanschlusseinheit ist über eine Steuerleitung mit einer Zentralventileinheit verbunden. Über Druckpunkte in der Fußanschlusseinheit beaufschlagt eine Druckkammer die Steuerleitung zur Ansteuerung der Zentralventileinheit. Um nach der Beugung des Kniegelenkes sicherzustellen, dass eine Streckung einfach zu realisieren ist, ist die Streckkammer über eine weitere hydraulische Leitung mit einem hydraulischen Federspeicher verbunden, der durch das Umlauföl von der Dämpferkammer beaufschlagt wird, um die in der Beugung gespeicherte Energie für die Bewegungsumkehr wieder freizusetzen. Der Federspeicher wirkt auf einen Kolben, der das Hydraulikfluid mit Druck beaufschlagt.

Die US 7 066 964 B2 betrifft ein Prothesenkniegelenk mit einer Rotationshydraulik. In einem Gehäuse in einem Oberteil wird über einen Frontallenker eines polyzentrischen Kniegelenkes ein Drehkolben verschwenkt. Eine Vorbringerunterstützung wird über einen federbelasteten Hebelmechanismus erreicht, die auf einen hinteren Lenker wirkt.

Die DE 195 06 426 C1 betrifft ein Bremskniegelenk für eine Beinprothese mit einem Gelenkoberteil, einem Gelenkunterteil, einer drehfesten mit einem Gelenkteil verbundenen Gelenkachse und einem ein Gelenkmittelteil bildenden Schwinghebel, der mit seinem streckseitigen Ende an einer parallel, ventral und distal zur Gelenkachse liegende Schwingachse festgelegt ist und mit seinem beugeseitigen Ende die Gelenkachse umschließt. Eine Bremseinrichtung wird über eine Fußbelastung gesteuert. Innerhalb des Bremskniegelenkes ist eine Rotationshydraulik angeordnet, bei der eine Ölleitung über einen Ventilkolben ganz oder teilweise verschließbar ist. Ein als Vorbringer dienendes Pleuel ist mit einem Ende an dem Gelenkoberteil und mit dem anderen Ende an einem in dem Gelenkunterteil angeordneten Federspanner angelenkt, der von einer Vorbringfeder beaufschlagt wird.

Die DE 10 2005 029 160 A1 betrifft eine hydraulische Kniegelenkprothese für eine Beinprothese mit einer Gelenkeinheit, einer Fußanschlusseinheit und einem beide Komponenten verbindenden Rohr, wobei die Gelenkeinheit eine Drehachse mit einer Dämpferkammer und einem darin bewegbaren Dämpferflügel beinhaltet, die von einer Hydrauliksteuerung angesteuert ist. Um nach einer Beugung des Kniegelenkes sicherzustellen, dass auch eine Streckung einfach zu realisieren ist, ist eine Streckkammer über eine hydraulische Leitung mit einem hydraulischen Federspeicher verbunden, der durch Umlauföl der Dämpferkammer beaufschlagt wird, um die bei der Beugung abgegebene Energie für die Bewegungsumkehr wieder freizusetzen.

Problematisch bei den Einrichtungen aus dem Stand der Technik ist, dass die Extensionsunterstützungen wartungsintensiv sind, Kleidungsstücke einklemmen können und bei Verschmutzung zu Verschleiß und gegebenenfalls zu einer Geräuschbildung neigen.

Aufgabe der vorliegenden Erfindung ist es, eine gegenüber dem Stand der Technik vereinfachte Konstruktion bereitzustellen, bei der der Verschleiß minimiert wird und eine sichere Handhabung gewährleistet ist.

Erfindungsgemäß wird diese Aufgabe durch ein orthopädietechnisches Gelenk mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Das erfindungsgemäße orthopädietechnische Gelenk, z.B. für Prothesen oder Orthesen, mit einem Oberteil, einem daran um eine Schwenkachse schwenkbar gelagerten Unterteil und einer Rotationshydraulik, die ein Gehäuse mit einer Kammer und einem darin schwenkbar gelagerten Schwenkkolben aufweist, der die Kammer in eine Flexionskammer und eine Extensionskammer unterteilt, die über zumindest ein Kanal hydraulisch miteinander verbunden sind, und mit einer Vorspanneinrichtung, die eine Verschwenkbewegung des Oberteils relativ zu dem Unterteil unterstützt, sieht vor, dass die Vorspanneinrichtung über eine Stütze direkt mit dem Schwenkkolben gekoppelt ist. Durch die direkte Kopplung der Stütze mit dem Schwenkkolben wird die Unterstützungskraft, insbesondere zur Unterstützung einer Vorbringbewegung oder eine Extension, unmittelbar auf den Schwenkkolben ausgeübt, der wiederum mit dem Oberteil oder dem Unterteil drehfest verbunden ist. Über die Kraftaufbringung auf den Schwenkkolben wird dadurch die jeweils beabsichtigte Verschwenkbewegung unterstützt. Die Anordnung der Stütze als mechanisches Festkörperbauteil an dem Schwenkkolben führt dazu, dass zumindest ein Teil der Stütze innerhalb des Hydraulikfluids geführt ist, wodurch ein Kontakt der Vorspanneinrichtung mit der Luft vermieden wird. Die Stütze, über die die Vorspannkraft auf den Schwenkkolben übertragen wird, befindet sich innerhalb des Nassbereiches der Hydraulik, sodass kein außerhalb der Rotationshydraulik angeordneter Vorbringer oder ein außerhalb der Hydraulik angeordnetes Kraftübertragungselement notwendig ist. Durch die Führung innerhalb der Hydraulik wird eine Geräuschentwicklung vermieden oder zumindest gedämpft. Der Verschleiß wird durch die Anordnung der Vorspann- oder Vorbringeinrichtung innerhalb des Hydraulikfluids, das in der Regel als ein Hydrauliköl ausgebildet ist, minimiert, da das Hydraulikfluid gleichzeitig den Kontakt mit Wasser abschirmt und darüber hinaus eine Schmierung sicherstellt.

Eine Weiterbildung der Erfindung sieht vor, dass die Stütze schwenkbar in oder an dem Schwenkkolben gelagert ist, wodurch sichergestellt werden kann, dass ein sehr hoher Beugewinkel des Oberteils relativ zu dem Unterteil um die Schwenkachse erreicht werden kann. Zur Übertragung von Druckkräften ist es vorteilhaft, wenn die Stütze knickstabil ausgebildet ist, wobei die Stütze nicht nur Druckkräfte von der Vorspanneinrichtung auf den Schwenkkolben übertragen kann, sondern in einer Variante der Erfindung auch Zugkräfte aufnehmen kann. Je nach Ausgestaltung der Vorspanneinrichtung ist es auch möglich, dass über die Stütze nur Zugkräfte auf den Schwenkkolben übertragen werden, wenn die Vorspanneinrichtung eine Zugfeder aufweist.

Die Stütze ist bevorzugt vollständig in dem Hydraulikfluid geführt, wodurch der Bauraum, der durch das Gelenk bereitgestellt wird, optimal ausgenutzt wird. Die Vorspanneinrichtung kann eine Feder aufweisen, auf der oder an der die Stütze gelagert ist. Die Feder ist insbesondere als eine Schrauben- oder Wendelfeder ausgebildet, die ebenfalls in dem Hydraulikfluid gelagert sein kann. Über die Anordnung eines Gleitstückes ist es möglich, eine optimierte Lagerung der Stütze auf der Feder zu erreichen, sodass die Federeigenschaften unabhängig von den Lagerungseigenschaften eingestellt werden können. So kann beispielsweise eine Wendelfeder oder Schraubenfeder verwendet werden, in deren Ende das Gleitstück eingesetzt wird, auf dem dann wiederum die Stütze Kräfte übertragend gelagert sein kann, insbesondere druckkraftübertragend gelagert sein kann. Ebenso ist es möglich, dass die Feder als Elastomerelement oder auch als eingekapseltes Luftkissen ausgebildet ist, auf dem das Gleitstück eine verbesserte Druckverteilung bewirkt. Bei einem Elastomerelement oder einem Druckkissen ist die hydraulische Konstruktion so gestaltet, dass die Hydraulikflüssigkeit oder das Öl verdrängt werden kann, beispielsweise in einen Ausgleichsbehälter, so dass innerhalb der Kammern kein Druckaufbau in dem Fluid durch die Vorspanneinrichtung erfolgt.

Eine Variante der Erfindung sieht vor, dass das Gleitstück in einer Buchse, insbesondere in einer geraden Buchse geführt ist. Die Buchse kann in das Gehäuse oder in das Oberteil beziehungsweise Unterteil eingeschraubt oder auf andere Art und Weise daran eingesetzt oder befestigt sein. Die Buchse steht in strömungstechnischer Verbindung mit der Kammer und stellt somit ein Teil des hydraulischen Systems dar. Somit befinden sich sowohl die Feder der Vorspanneinrichtung als auch das Gleitstück und die Stütze innerhalb der Hydraulikfluid, sodass sämtliche bewegten Komponenten der Vorspanneinrichtung und der Einrichtung zur Unterstützung der Verschwenkbewegung innerhalb des Hydraulikfluids gelagert sind. Hierdurch wird insgesamt der Verschleiß auch des Gleitstückes und die Geräuschentwicklung verringert. Darüber hinaus kann durch die direkte Anbindung der Vorspanneinrichtung und der Stütze an den Schwenkkolben die Anzahl der Bauteile auf ein Minimum begrenzt werden. Es werden keine gesonderten Lagerstellen mit der Notwendigkeit einer separaten Schmierung oder einer aufwendigen Gleitlagerung benötigt.

In dem Gleitstück kann zumindest eine Ausnehmung und/oder ein Durchlass für das Hydraulikfluid angeordnet oder ausgebildet sein, damit das Hydraulikfluid aus der Kammer, in dem sich der Schwenkkolben befindet, in einen Raum gelangen kann, in den das Gleitstück hinein und wieder herausbewegt wird, wenn der Schwenkkolben bewegt wird. Die zumindest eine Ausnehmung und/oder Dämpfung ist so groß bemessen, dass keine nennenswerte Dämpfung erzeugt wird. Die durch den Strömungswiderstand des Gleitstückes erzeugte Dämpfung ist grundsätzlich unerwünscht, da sie unbeeinflussbar gegen die Verschwenkbewegung des Rotationskolbens arbeitet. Bevorzugt ist die Ausgestaltung des Gleitstückes dergestalt, dass eine minimale, vernachlässigbare Dämpfung erzeugt wird.

Die Vorspanneinrichtung ist bevorzugt so ausgebildet, dass durch sie keine nennenswerten Kräfte auf das Fluid übertragen werden, sondern dass die Vorspanneinrichtung nur oder nahezu ausschließlich über die Stütze auf den Schwenkkolben wirkt. Dadurch werden hydraulische Effekte und Beeinflussungen der Verschwenkbewegung durch die Vorspanneinrichtung voneinander getrennt, so dass die bei der Abstimmung des Gelenkes keine Wechselwirkungen zu beachten sind.

In dem Schwenkkolben kann eine Ausnehmung ausgebildet sein, in der die Stütze aufgenommen ist. Durch die Ausnehmung innerhalb des Schwenkkolbens kann der Schwenkwinkel weiter vergrößert werden, da die Stütze bei einer Verschwenkung in eine Extremlage in die Kontur des Schwenkkolbens eintauchen kann, wobei die Ausnehmung nicht durchgängig ausgebildet ist, sodass der Schwenkkolben weiterhin die Extensionskammer von der Flexionskammer trennt.

Die Stütze kann eine S-förmige Kontur aufweisen, um es zu ermöglichen, dass die Endlagen des Schwenkkolbens möglichst weit entfernt voneinander liegen, um eine maximale Einbeugung des Gelenkes zu ermöglichen. Durch die Ausgestaltung des Schwenkkolbens mit einer Ausnehmung und die S-förmige Kontur der Stütze können Beugewinkel von mehr als 150° verwirklicht werden.

In einer Variante der Erfindung ist es vorgesehen, dass der Kopplungspunkt des Schwenkkolbens mit der Stütze in den Endlagen des Schwenkkolbens auf unterschiedlichen Seiten der Schwenkachsen liegt, wodurch sich unter anderem auch erreichen lässt, dass bei einer Vorspannung der Stütze durch die Vorspanneinrichtung in eine der Endlagen eine Sicherung in der jeweiligen Endlage ergibt, sodass beispielsweise eine erhöhte Kraft aufgewendet werden muss, um aus einer maximal flektierten oder maximal extendierten Stellung in die Extension oder Flexion zu gelangen.

Um einen Volumentransport von der Extensionskammer in die Flexionskammer und umgekehrt zu ermöglichen, ist zwischen den Kammern ein Kanal angeordnet, durch den Hydraulikfluid strömen kann, wenn der Schwenkkolben bewegt wird. Bevorzugt ist der Kanal in demjenigen Gehäuse ausgebildet, in dem auch die Kammer mit dem darin gelagerten Schwenkkolben ausgebildet ist. Der Kanal kann auch separat als Leitung ausgebildet sein. In dem Kanal kann eine bevorzugt einstellbare Drossel angeordnet sein, über die der Verschwenkwiderstand der Gelenkeinrichtung aufgrund eines verstellbaren hydraulischen Widerstandes verändert werden kann. Die Drossel kann einen permanenten Widerstand bereitstellen, ebenso ist es möglich, durch eine entsprechende hydraulische Gestaltung unterschiedliche Widerstände in Extensionsrichtung und Flexionsrichtung vorzusehen. Ebenso kann durch ein Bypassventil die Verschwenkbewegung in eine Richtung nahezu ohne hydraulischen Widerstand ausgeführt werden. Ebenfalls ist es möglich, über eine Sensoreinrichtung und eine Steuereinrichtung sowie eine Verstelleinrichtung ein computergesteuertes Kniegelenk mit der Vorspanneinrichtung zu koppeln, wie sie oben beschrieben ist.

Das orthopädietechnische Gelenk ist insbesondere für eine Prothese oder Orthese geeignet, ist insbesondere als Prothesengelenk oder Orthesengelenk ausgebildet und ist in einer Ausgestaltung der Erfindung als ein Prothesenkniegelenk ausgebildet.

Nachfolgend wird ein Ausführungsbeispiel der Erfindung anhand der beigefügten Figuren näher erläutert. Es zeigen:
- Figur 1-: eine Seitenansicht eines künstlichen Gelenks in Gestalt eines Prothesenkniegelenks;
- Figur 2-: eine Teilschnittdarstellung des Prothesenkniegelenks gemäß Figur 1;
- Figur 3-: eine Detailansicht in maximal extendierter Stellung; sowie
- Figur 4-: eine Darstellung der Figur 3 in maximal flektierter Stellung.

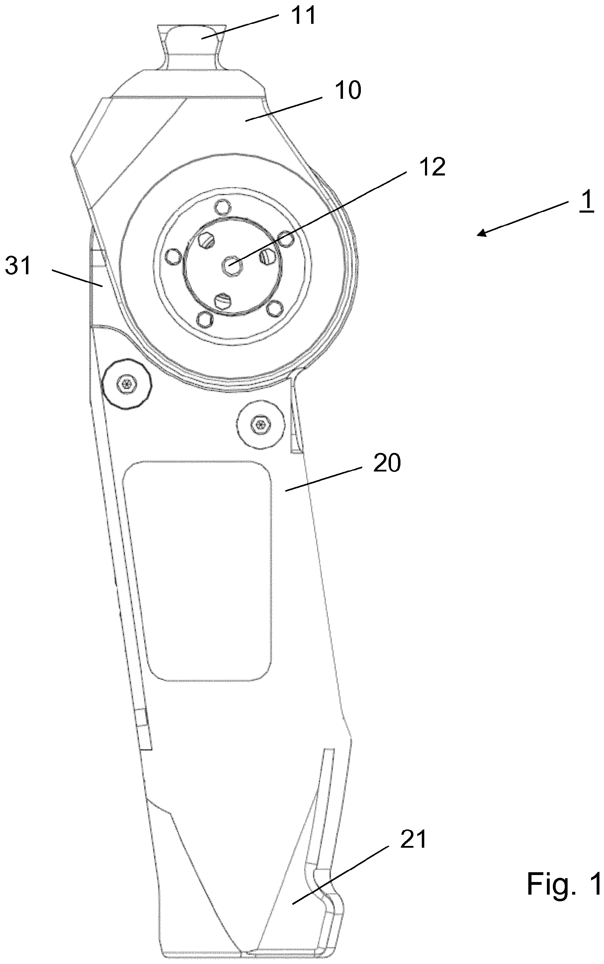

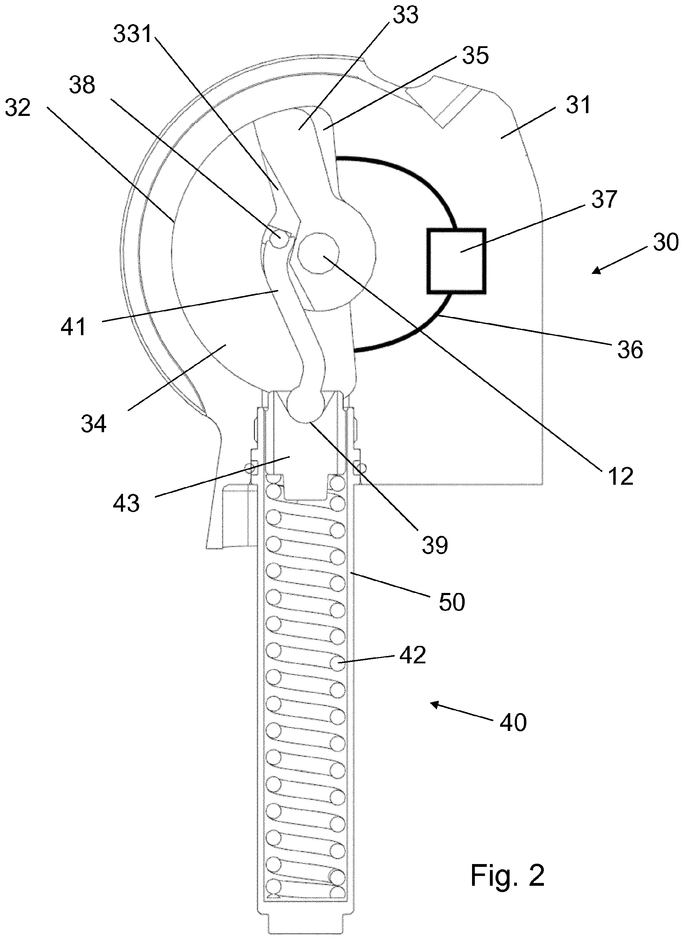

In Figur 1 ist in einer Seitenansicht ein orthopädietechnisches Gelenk in Gestalt eines Exoprothesenkniegelenkes 1 dargestellt, das ein Oberteil 10 mit oberen Anschlussmitteln 11 in Gestalt eines Pyramidenadapters aufweist. An dem oberen Anschlussmittel 11 kann ein Prothesenschaft zur Aufnahme eines Oberschenkelstumpfes befestigt werden. Das Oberteil 10 ist um eine Schwenkachse 12 schwenkbar um ein Unterteil 20 gelagert, an dessen distalem Ende eine Aufnahme 21 für ein Unterschenkelrohr ausgebildet ist. Innerhalb des Unterteils 20 ist ein Gehäuse 31 ausgebildet oder angeordnet, in dem eine Rotationshydraulik untergebracht ist. Innerhalb des Unterteils 20 können weitere Komponenten der Rotationshydraulik angeordnet sein, was in Zusammenhang mit der Figur 2 erläutert wird.

Figur 2 zeigt in einer Schnittdarstellung einen Teil des Prothesenkniegelenkes 1 gemäß Figur 1 mit einem Gehäuse 30, in dem eine Kammer 32 ausgebildet ist, in der ein Schwenkkolben 33 schwenkbar um die Schwenkachse 12 gelagert ist. Das Prothesenkniegelenk 1 ist als monozentrisches Kniegelenk ausgebildet, und der Schwenkkolben 33 ist drehfest mit dem Oberteil 10 verbunden. In der dargestellten Position gemäß Figur 2 befindet sich der Schwenkkolben 33 in der Extensionsendlage, in der das Prothesenkniegelenk eine maximale Streckung erreicht hat. In dem dargestellten Ausführungsbeispiel wird ein Extensionsanschlag durch externe Puffer in dem Oberteil verwirklicht, die in der Extremstellung auftretenden Kräfte werden auf das Hydraulikteil übertragen. Der Endanschlag in dem Gelenk wird nicht über den Schwenkkolben 33 ausgebildet, um unter anderem diesen nicht für die hohen mechanischen Kräfte auslegen zu müssen, die in einem Endanschlag auftreten können. Der Schwenkkolben 33 unterteilt die Kammer 20 in eine Extensionskammer 35 und eine Flexionskammer 34. Wird das Oberteil 10 relativ zu dem Unterteil 20 in Flexionsrichtung verschwenkt, wird aufgrund der drehstarren Kopplung des Schwenkkolbens 30 mit dem Oberteil 10 der Schwenkkolben 33 entgegen dem Uhrzeigersinn innerhalb der Kammer 32 verschwenkt. Hydraulikfluid, das in der Kammer 32 befindlich ist, wird von der Flexionskammer durch den Kanal 36 in dem Gehäuse 30 und durch eine Drosseleinrichtung 37 in die Extensionskammer 35 bewegt. Die Drossel 37 kann verstellbar oder einstellbar ausgebildet sein. Eine Verstellbarkeit kann durch eine Computersteuerung auf Basis von Sensoren erfolgen. Alternativ kann die Drossel 37 dauerhaft auf den jeweiligen Nutzer eingestellt werden. Zur Verstellung kann über einen Zugang von außen die Drossel 37 manuell und dauerhaft verstellt werden.

An dem Schwenkkolben 33 ist direkt eine Stütze 41 in Gestalt eines S-förmigen Stabes gelagert. Die Stütze 41 weist einen schwenkkolbenseitigen Kopplungspunkt 38 auf. Der Kopplungspunkt 38 ist so ausgebildet, dass die Stütze 41 um den Kopplungspunkt 38 verschwenkt werden kann. Dadurch ist es möglich, dass bei einer Flexionsbewegung, also einer Verschwenkung des Schwenkkolbens 33 entgegen dem Uhrzeigersinn, die Stütze 41 auf dem Kopplungspunkt 38 abgleiten kann, wenn der Kopplungspunkt 38 eine Teilkreisbewegung ausführt. Die Lagerung in dem Kopplungspunkt 38 kann druckkraftübertragend und zugkraftübertragend ausgebildet sein.

Das andere, gerundete Ende der Stütze 41 ist einem Gleitstück 43 gelagert, das eine Vertiefung aufweist, in der das dem Schwenkkolben 33 abgewandte Ende der Stütze 41 eingesetzt ist. Auch hier ist eine Rotationsbewegung im Lagerungspunkt 39 auf dem Gleitstück 43 möglich, um die bei der Verschwenkung des Schwenckolbens 33 auftretenden Schwenkbewegung der Stütze 41 sowohl in dem gleitstückseitigen Lagerungspunkt 39 ausführen zu können.

Das Gleitstück 43 ist in einer Buchse 50 geführt, die in das Gehäuse 30 eingeschraubt ist. Innerhalb der Buchse 50 ist eine Schraubenfeder 42 angeordnet, sodass sich aus der Kombination aus der Feder 42, dem Gleitstück 43 und der Stütze 41 eine Vorspanneinrichtung 40 ergibt, über die Druckkräfte von einer komprimierten Feder 42 unmittelbar auf den Schwenkkolben 33 übertragen werden können.

Das Gleitstück 43 ragt in die Kammer 32 hinein, wenn sich der Schwenkkolben 33 in der dargestellten Stellung befindet.

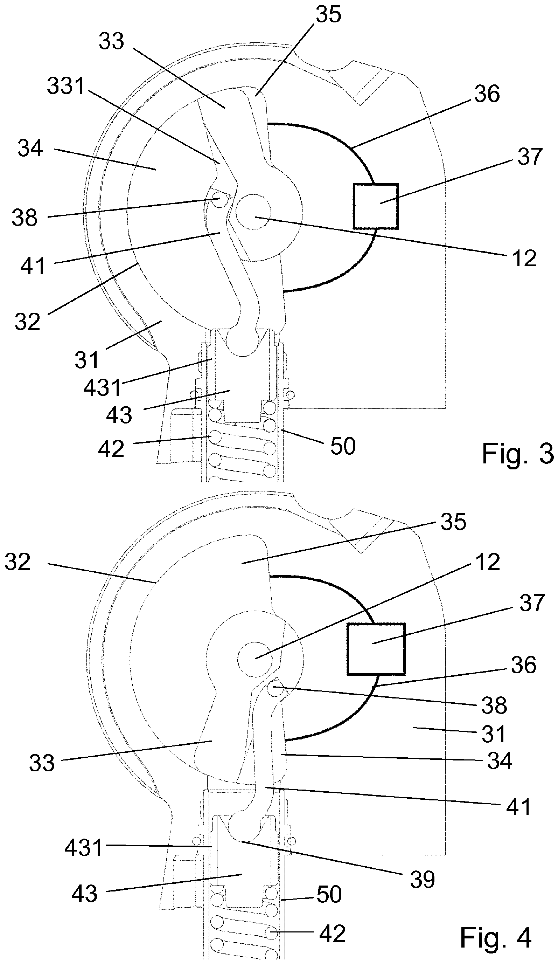

In der Figur 3 ist die Stellung gemäß Figur 2 in einer Teilansicht gezeigt. An der Außenseite des Gleitstücks 43 sind Durchlässe, Ausnehmungen oder Kanäle 431 beispielsweise in Gestalt von Nuten ausgebildet, durch die die Hydraulikflüssigkeit aus der Kammer 32 in die Buchse 50 hinein- und herausfließen kann. Somit sind sowohl die Buchse 50 als auch die Kammer 32 und der Verbindungskanal 36 mit dem Hydraulikfluid gefüllt. Sowohl die Feder 42 als auch das Gleitstück 43 und die Stütze 41 befinden sich innerhalb des Hydraulikfluids, sodass dieses gleichzeitig eine Schmierfunktion, eine Geräuschdämpfungsfunktion sowie eine Abkapselung der bewegten Komponenten der Vorspanneinrichtung 40 gegenüber äußeren Einflüssen übernimmt. Das Gleitstück 43 ist mit den Durchlässen 431 so ausgebildet, dass kein oder nahezu kein hydraulischer Widerstand der Verschwenkbewegung entgegengesetzt wird.

In der Figur 3 ist zu erkennen, dass der Schwenkkolben 33 eine Ausnehmung 331 aufweist, die im Bereich der Lagerung der Stütze 41 an dem Schwenkkolben 33 ausgebildet ist. Die Ausnehmung 331 kann als Nut ausgebildet sein und erstreckt sich nicht über die vollständige Breite des Schwenkkolbens 33. Die Ausnehmung 331 dient unter anderem dazu, in einer maximal flektierten Stellung zu verhindern, dass die Stütze 41 mit der Kammerinnenwand 32 kollidiert, so dass ein möglichst großer Verschwenkwinkel erreicht werden kann. Die nutartige Ausnehmung 331 erstreckt sich nicht über die gesamte Höhe des Schwenkkolbens 33 und ist bevorzugt nur so breit, dass sich die Stütze 41 darin bewegen kann. Die Ausnehmung 331 dient dazu, dass die Stütze 31 trotz der exzentrischen Anordnung des Lagerpunktes 38 an dem Kolben möglichst nahe an der Schwenkachse 12 annähern kann, wodurch ein kompakter Bauraum erzielt werden kann. Die Ausnehmung 331 erstreckt sich beabstandet von der der Kammerwand 32 zugewandten Spitze des Schwenkkolbens 33 bis zum gegenüberliegenden Fußbereich auf der der Schwenkachse 12 abgewandten Seite des Schwenkkolbens 33.

Wird der Schwenkkolben 33 gegen den Uhrzeigersinn verdreht, wenn das Oberteil 10 flektiert wird, wandert der Koppelpunkt 38 der Stütze 41 mit dem Schwenkkolben 33 auf einer Kreisbahn um die Schwenkachse 12. Dabei wird der Koppelpunkt 38 und damit auch die Stütze 41 um den unteren Lagerungspunkt 39 entgegen dem Uhrzeigersinn verschwenkt, bis die maximale seitliche Auslenkung erreicht wird. Bei einer weiteren Rotation gegen den Uhrzeigersinn verschwenkt der Koppelpunkt 38 unterhalb der Schwenkachse, wobei durch die Kreisbewegung des Koppelpunktes 38 die Stütze 41 als druckkraftübertragendes Bauteil aus Metall oder einem formstabilen Kunststoff auch eine Bewegung nach unten ausführt, sodass das Gleitstück 43 entgegen der Federkraft der Feder 42 in die Buchse 50 eingeschoben wird. Das Gleitstück 43 wird maximal in die Buchse 50 hineingeschoben, wenn der Kopplungspunkt 38 senkrecht unterhalb der Schwenkachse 12 liegt.

In der Figur 4 ist die nahezu maximal flektierte Stellung des Kniegelenks 1 dargestellt, in der die Extensionskammer 35 ein angenähert maximales Volumen aufweist, während die Flexionskammer 34 ein angenähert minimales Volumen aufweist. Der Kopplungspunkt 38 befindet sich auf der rechten Seite der Schwenkachse 12, also auf der gegenüberliegenden Seite der Schwenkachse 12 verglichen mit der maximal extendierten Stellung gemäß Figur 3. Aufgrund der s-förmigen Kontur der Stütze 41 und der Ausnehmung 331 in dem Schwenkkolben 33 ist es möglich, dass der Schwenkkolben 33 um nahezu 180 Grad um die Schwenkachse 12 verschwenkbar ist. Die Feder 42 befindet sich in einem vorgespannten Zustand. Aufgrund der rechts der Schwenkachse 12 befindlichen Position, drückt die Feder 42 über das Gleitstück 43 und die Stütze 41 den Kolben 43 weiter in die Flexionsstellung, da die Kraftwirkungslinie eine entsprechende Drehung des Schwenkkolbens 33 entgegen dem Uhrzeigersinn unterstützt. Wird das Gelenk 1 wieder extendiert, erfolgt eine Extensionsunterstützung erst, nachdem der Kopplungspunkt 38 jenseits der Senkrechten, die durch die Drehachse 12 verläuft, bewegt worden ist. Die durch die sich entspannende Feder 42 ausgeübte Druckkraft der an dem Schwenkkolben 33 direkt gelagerten Stütze 41 bewirkt eine Extensionsunterstützung und bei einem Prothesenkniegelenk eine Vorbringerbewegung des Unterteils 20.

Grundsätzlich ist es auch möglich, eine solche Gelenkeinrichtung oder ein solches Gelenk 1 in eine Orthese einzubauen. Auch die Anwendung an anderen Gelenkstellen, beispielsweise an einem Ellenbogengelenk, ist möglich. Statt einer Extensionsunterstützung kann durch eine entsprechende Anordnung der Vorspanneinrichtung 40 auch eine Flexionsunterstützung erfolgen, je nachdem, welche Orientierung sowohl die Feder 42 als auch die Anlenkung der Stütze 41 haben.

Aufgrund der ständig wirkenden Vorspannung durch die Feder 42 muss die Lagerung der Stütze 41 an dem oberen Koppelpunkt 38 und dem unteren Lagerungspunkt 39 nicht zugkraftübertragend sein. Grundsätzlich ist es auch möglich, dass beispielsweise durch eine Steckachse die Stütze 41 schwenkbar an dem Drehkolben 33 zugkraftübertragend und druckkraftübertragend gelagert ist. Gleiches gilt für die Lagerung auf dem Gleitstück 43.

Neben einer geradlinigen Ausgestaltung der Buchse 50 kann diese auch gekrümmt ausgebildet sein. Statt einer Schraubenfeder 42 können abweichende Federeinrichtungen oder Kraftspeichereinrichtungen vorgesehen sein, beispielsweise Tellerfedern, Schraubentellerfedern oder ähnliches. Die Buchse 50 befindet sich innerhalb des Unterteils 20. Die Buchse 50 kann in das Gehäuse eingeschraubt sein, sodass eine leichte Montage der gesamten Vorspanneinrichtung 40 erfolgen kann. Dadurch ist es möglich, unterschiedliche Federn 42 einzusetzen oder eine entsprechende Gelenkeinrichtung nachträglich mit einer Vorspanneinrichtung 40 auszurüsten. Ohne eine Vorspanneinrichtung 40 wird der Zugang zu der Kammer 32 einfach über einen Stopfen verschlossen.

## Patentansprüche

1. Orthopädietechnisches Gelenk mit einem Oberteil (10), einem daran um eine Schwenkachse (12) schwenkbar gelagerten Unterteil (20) und einer Rotationshydraulik (30), die ein Gehäuse (31) mit einer Kammer (32) und einem darin schwenkbar gelagerten Schwenkkolben (33) aufweist, der die Kammer (32) in eine Flexionskammer (34) und eine Extensionskammer (35) unterteilt, die über zumindest einen Kanal (36) hydraulisch miteinander verbunden sind, und mit einer Vorspanneinrichtung (40), die eine Verschwenkbewegung des Oberteils (10) relativ zu dem Unterteil (20) unterstützt, **dadurch gekennzeichnet, dass** die Vorspanneinrichtung (40) über eine Stütze (41) direkt mit dem Schwenkkolben (33) gekoppelt ist.

2. Gelenk nach Anspruch 1, **dadurch gekennzeichnet, dass** die Stütze (41) schwenkbar in oder an dem Schwenkkolben (33) gelagert ist.

3. Gelenk nach Anspruch 1 oder 2, **dadurch gekennzeichnet, dass** die Stütze (41) knickstabil ausgebildet ist.

4. Gelenk nach einem der voranstehenden Ansprüche, **dadurch gekennzeichnet, dass** die Stütze (41) Zug- und/oder Druckkräfte von der Vorspanneinrichtung (40) auf den Schwenkkolben (33) überträgt.

5. Gelenk nach einem der voranstehenden Ansprüche, **dadurch gekennzeichnet, dass** die Stütze (41) in dem Hydraulikfluid geführt ist.

6. Gelenk nach einem der voranstehenden Ansprüche, **dadurch gekennzeichnet, dass** die Vorspanneinrichtung (40) eine Feder (42) aufweist, auf oder an der die Stütze (41) gelagert ist.

7. Gelenk nach Anspruch 6, **dadurch gekennzeichnet, dass** die Stütze (41) auf oder an einem Gleitstück (43) gelagert ist, das zwischen der Feder (42) und der Stütze (41) angeordnet ist.

8. Gelenk nach Anspruch 7, **dadurch gekennzeichnet, dass** das Gleitstück (43) in einer Buchse (50) geführt ist, die in strömungstechnischer Verbindung mit der Kammer (32) steht.

9. Gelenk nach einem der Ansprüche 7 oder 8, **dadurch gekennzeichnet, dass** in oder an dem Gleitstück (43) zumindest eine Ausnehmung und/oder ein Durchlass (431) für das Hydraulikfluid angeordnet ist.

10. Gelenk nach einem der voranstehenden Ansprüche, **dadurch gekennzeichnet, dass** in dem Schwenkkolben (33) eine Ausnehmung (331) ausgebildet ist, in der die Stütze (41) aufgenommen ist.

11. Gelenk nach einem der voranstehenden Ansprüche, **dadurch gekennzeichnet, dass** die Stütze (41) eine S-förmige Kontur aufweist.

12. Gelenk nach einem der voranstehenden Ansprüche, **dadurch gekennzeichnet, dass** der Kopplungspunkt (38) des Schwenkkolbens (33) mit der Stütze (41) in den Endlagen des Schwenkkolbens (33) auf unterschiedlichen Seiten der Schwenkachse (12) liegt.

13. Gelenk nach einem der voranstehenden Ansprüche, **dadurch gekennzeichnet, dass** der Kanal (36) in dem Gehäuse (31) ausgebildet ist.

14. Gelenk nach einem der voranstehenden Ansprüche, **dadurch gekennzeichnet, dass** in dem Kanal (36) eine Drossel (37) angeordnet ist.

15. Gelenk nach einem der voranstehenden Ansprüche, **dadurch gekennzeichnet, dass** das Gelenk für eine Prothese oder Orthese geeignet ist und insbesondere als ein Prothesengelenk oder ein Orthesengelenk, insbesondere ein Prothesenkniegelenk ausgebildet ist.

## Claims

1. An orthopedic joint, comprising an upper part (10), a lower part (20) mounted on the upper part (10) so as to pivot about a pivot axis (12), and a rotation hydraulics unit (30), which has a housing (31) with a chamber (32) and with a pivot piston (33) which is mounted pivotably in the latter and divides the chamber (32) into a flexion chamber (34) and an extension chamber (35), said chambers being connected hydraulically to each other via at least one channel (36), and with a pretensioning device (40) which assists a pivoting movement of the upper part (10) relative to the lower part (20), **characterized in that** the pretensioning device (40) is coupled directly to the pivot piston (33) via a support (41).

2. The joint as claimed in claim 1, **characterized in that** the support (41) is mounted pivotably in or at the pivot piston (33).

3. The joint as claimed in claim 1 or 2, **characterized in that** the support (41) is stable against buckling.

4. The joint as claimed in one of the preceding claims, **characterized in that** the support (41) transmits tensile and/or compressive forces from the pretensioning device (40) to the pivot piston (33).

5. The joint as claimed in one of the preceding claims, **characterized in that** the support (41) is guided in the hydraulic fluid.

6. The joint as claimed in one of the preceding claims, **characterized in that** the pretensioning device (40) has a spring (42), on or at which the support (41) is mounted.

7. The joint as claimed in claim 6, **characterized in that** the support (41) is mounted on or at a slide piece (43), which is arranged between the spring (42) and the support (41).

8. The joint as claimed in claim 7, **characterized in that** the slide piece (43) is guided in a bushing (50), which is connected fluidically to the chamber (32).

9. The joint as claimed in either of claims 7 and 8, **characterized in that** at least one recess and/or a passage (431) for the hydraulic fluid is arranged in or at the slide piece (43).

10. The joint as claimed in one of the preceding claims, **characterized in that** a recess (331), in which the support (41) is received, is formed in the pivot piston (33).

11. The joint as claimed in one of the preceding claims, **characterized in that** the support (41) has an S-shaped contour.

12. The joint as claimed in one of the preceding claims, **characterized in that** the coupling point (38) of the pivot piston (33) to the support (41) lies on different sides of the pivot axis (12) in the end positions of the pivot piston (33).

13. The joint as claimed in one of the preceding claims, **characterized in that** the channel (36) is formed in the housing (31).

14. The joint as claimed in one of the preceding claims, **characterized in that** a throttle (37) is arranged in the channel (36).

15. The joint as claimed in one of the preceding claims, **characterized in that** the joint is suitable for a prosthesis or orthosis and is in particular designed as a prosthetic joint or an orthotic joint, in particular a prosthetic knee joint.

## Revendications

1. Articulation orthopédique comprenant une partie supérieure (10), une partie inférieure (20) montée sur celle-ci de manière à pouvoir pivoter autour d'un axe de pivotement (12), et un système hydraulique rotatif (30) qui présente un boîtier (31) ayant une chambre (32) et un piston pivotant (33) monté dans celle-ci de manière à pouvoir pivoter, lequel subdivise la chambre (32) en une chambre de flexion (34) et en une chambre d'extension (35) qui sont reliées hydrauliquement l'une à l'autre par au moins un canal (36), et comportant un dispositif de précontrainte (40) qui assiste un mouvement de pivotement de la partie supérieure (10) par rapport à la partie inférieure (20),
**caractérisée en ce que** le dispositif de précontrainte (40) est couplé directement au piston pivotant (33) par un support (41).

2. Articulation selon la revendication 1,
**caractérisée en ce que** le support (41) est monté dans ou sur le piston pivotant (33) de manière à pouvoir pivoter.

3. Articulation selon la revendication 1 ou 2,
**caractérisée en ce que** le support (41) est réalisé de façon résistante au flambage.

4. Articulation selon l'une des revendications précédentes,
**caractérisée en ce que** le support (41) transmet les efforts de traction et/ou de compression du dispositif de précontrainte (40) au piston pivotant (33).

5. Articulation selon l'une des revendications précédentes,
**caractérisée en ce que** le support (41) est guidé dans le fluide hydraulique.

6. Articulation selon l'une des revendications précédentes,
**caractérisée en ce que** le dispositif de précontrainte (40) comprend un ressort (42) sur lequel ou au niveau duquel est monté le support (41).

7. Articulation selon la revendication 6,
**caractérisée en ce que** le support (41) est monté sur ou au niveau d'une pièce coulissante (43) qui est disposée entre le ressort (42) et le support (41).

8. Articulation selon la revendication 7,
**caractérisée en ce que** la pièce coulissante (43) est guidée dans une douille (50) qui est en communication fluidique avec la chambre (32).

9. Articulation selon l'une des revendications 7 ou 8,
**caractérisée en ce qu'**au moins un évidement et/ou un passage (431) pour le fluide hydraulique est disposé dans ou sur la pièce coulissante (43).

10. Articulation selon l'une des revendications précédentes,
**caractérisée en ce qu'**un évidement (331) est ménagé dans le piston pivotant (33), dans lequel est reçu le support (41).

11. Articulation selon l'une des revendications précédentes,
**caractérisée en ce que** le support (41) présente un contour en forme de S.

12. Articulation selon l'une des revendications précédentes,
**caractérisée en ce que** le point de couplage (38) du piston pivotant (33) au support (41) se trouve sur différents côtés de l'axe de pivotement (12) dans les positions extrêmes du piston pivotant (33).

13. Articulation selon l'une des revendications précédentes,
**caractérisée en ce que** le canal (36) est formé dans le boîtier (31).

14. Articulation selon l'une des revendications précédentes,
**caractérisée en ce qu'**un étrangleur (37) est disposé dans le canal (36).

15. Articulation selon l'une des revendications précédentes,
**caractérisée en ce que** l'articulation convient pour une prothèse ou une orthèse et est en particulier réalisée sous forme d'articulation prothétique ou d'articulation orthétique, en particulier sous forme d'articulation prothétique du genou.
